# EUROPÄISCHE PATENTSCHRIFT

(11) **EP 1 023 075 B1**
(45) Veröffentlichungstag und Bekanntmachung des Hinweises auf die Patenterteilung: **20.12.2006**
(21) Anmeldenummer: 98959738.0
(22) Anmeldetag: 13.10.1998
(51) Int. Cl.: A61K 31/675, A61K 47/02, A61K 9/08

(54) **FLÜSSIGE DARREICHUNGSFORMEN OXAZAPHOSPHORINHALTIGER PHARMAZEUTISCHER PRODUKTE**
LIQUID PRESENTATIONS OF OXAZAPHOSPHORINE-CONTAINING PHARMACEUTICAL PRODUCTS
FORMES GALENIQUES LIQUIDES DE PRODUITS PHARMACEUTIQUES A BASE D'OXAZAPHOSPHORINE

(30) Priorität: 13.10.1997 DE 19745246
(43) Veröffentlichungstag der Anmeldung: 02.08.2000
(73) Patentinhaber: STADA ARZNEIMITTEL AG, 61118 Bad Vilbel (DE)
(72) Erfinder: BAYER, Matthias, D-22397 Hamburg (DE)
(74) Vertreter: Hamm, Volker
(86) Internationale Anmeldenummer: PCT/DE1998/003052
(87) Internationale Veröffentlichungsnummer: WO 1999/018973

(56) Entgegenhaltungen:
- EP-A- 0 254 902
- EP-A- 0 538 858
- WO-A-95/07083
- WO-A-99/18973
- US-A- 5 036 060
- US-A- 5 227 374
- US-A- 5 413 995
- MUNOZ M ET AL.: "Stability of ifosfamide in 0.9% sodium chloride solution or water for injection in a portable i.v. pump cassette" AMERICAN JOURNAL OF HOSPITAL PHARMACISTS, Bd. 49, 1992, Seiten 1137-71139, XP002103761
- LOEHRER P J ET AL: "Salvage therapy in recurrent germ cell cancer: Ifosfamide and cisplatin plus either vinblastine or etoposide" ANNALS OF INTERNAL MEDICINE, Bd. 109, Nr. 1, 1. Oktober 1988, Seiten 540-546, XP002083105

## Beschreibung

Die vorliegende Erfindung betrifft die Verwendung einer Chloridionen bildenden Verbindung zur Stabilisierung eines Oxazaphosphorins in wäßrigen Arzneilösungen.

Ferner betrifft die Erfindung optimierte flüssige Darreichungsformen oxazaphosporinhaltiger pharmazeutischer Produkte mit gegenüber bestehenden flüssigen und festen Zubereitungen verbesserter Lagerstabilität.

Oxazaphosphorine sind Stickstofflostderivate. Sie gehören zur Gruppe der alkylierenden Cytostatika und werden zur Behandlung von Tumorerkrankungen eingesetzt. Wäßrige Oxazaphosphorinlösungen, insbesondere von Ifosfamid und Cyclophosphamid, sind besonders vorteilhaft für die intravenöse Injektion als auch vernebelt zur Inhalation für Mensch und Tier bei der Behandlung verschiedener Tumorerkrankungen.

Ein therapeutisch bedeutendes Oxazaphosphorin, für das das erfindungsgemäße Verfahren beziehungsweise die entsprechenden Lösungen insbesondere in Frage kommen, ist zum Beispiel Ifosfamid. Die chemische Bezeichnung für Ifosfamid ist 2-[Bis-(2-chlorethyl)-amino]-tetrahydro-2H-1,3,2-oxazaphosphorin-2-oxid (C₇H₁₅Cl₂N₂O₂P, Mᵣ261,09).

Die therapeutische Anwendung von Ifosfamid und Cyclophosphamid erfolgt durch perorale und parenterale Zubereitungsformen. Die parenterale Applikation erfolgt vorwiegend in Form von Infusionen. Infolge der begrenzten Stabilität der Oxazaphosphorine in wäßriger Lösung sind lagerfähige gebrauchsfertige wäßrige Injektionslösungen nicht bekannt. Bevorzugte parenterale Zubereitungsformen bestehen derzeit vielmehr aus Injektionsflaschen, die unterschiedliche Wirkstoffdosen als Trockensubstanz (Sterilkristallisat, Lyophilisat) enthalten, aus denen die Injektionslösung unmittelbar vor der Applikation hergestellt wird.

Im Falle des Ifosfamids besteht die Trockensubstanz für die parenterale Anwendung beispielsweise aus kristallinem Ifosfamid. Der Einsatz der kristallinen Substanz ist gegenüber einer gebrauchsfertigen Injektionslösung jedoch mit erheblichen Nachteilen verbunden. So muß bereits bei der Gewinnung des Wirkstoffs, beispielsweise bei der Kristallisation, beim Abzentrifugieren der Kristalle, beim Trocknen und Mahlen, steril und unter aseptischen Bedingungen gearbeitet werden. Schließlich muß das fertige Gemisch unter sterilen Kautelen exakt dosiert abgefüllt werden.

Dieses aufwendige Verfahren ist ständig der Gefahr partikulärer und mikrobieller Kontaminationen ausgesetzt. Darüber hinaus läßt sich bei aller Sorgfalt in der Herstellung die Bildung verschieden großer Kristalle nicht vermeiden, so daß die Anfertigung von Cyclophosphamidlösungen bzw. Ifosfamidlösungen mit Trockensubstanz in Injektionsflaschen einen erheblichen Zeitaufwand erfordert. Vor allem in Krankenanstalten führt dies zu einer häufig nicht zumutbaren Mehrbelastung des Krankenpflegepersonals.

Sterilkristallisate der Oxazaphosphorine, insbesondere von Ifosfamid und Cyclophosphamid, beginnen bei längerer Lagerung unter Gelbfärbung zu sintern. Diese Verdichtung der Trockensubstanz bedeutet eine deutliche Abnahme der Löslichkeit. Eine Gelbfärbung der Trockensubstanz bedeutet einen deutlichen Wirkstoffverlust. Im allgemeinen ist dann eine Verwendung der Trockensubstanz nicht mehr möglich.

Die Verfahren zur Herstellung der Trockensubstanz in der Form des Lyophilisates sind gegenüber dem Sterilkristallisat weniger aufwendig. Lyophilisate sind durch den Vorteil gekennzeichnet, daß unmittelbar vor der Abfüllung der gefrierzutrocknenden Lösung sterilfiltriert und somit die Gefahr partikulärer und mikrobieller Kontaminationen vermieden werden kann. Durch den Einsatz von gerüstbildenden Hilfsstoffen, beispielsweise Lactose (DE 195 40 132), Hexit (EP 265 812, EP 401 894), Mannit (US 4 537 883), Galactit (EP 251 657), Alanin (EP 394 045), Kochsalz (WO 95/07083, US 5 036 060), Harnstoff (EP 538 858) oder Natriumbicarbonat (US 522 737, US 5 130 305), kann durch die Lyophilisation eine sehr homogene Trockensubstanz erzeugt werden.

Diese Lyophilisate haben den Vorteil, daß ein geringerer Zeitaufwand für die Anfertigung der Lösungen aus der Trockensubstanz erforderlich ist. Der Nachteil einer Sinterung des Wirkstoffs kann durch das Lyophilisat weitgehend vermieden, der Nachteil einer Gelbfärbung der Trockensubstanz jedoch nicht vollständig ausgeschlossen werden.

Der Einsatz der Trockensubstanzen ist gegenüber einer unmittelbar gebrauchsfertigen Injektionslösung jedoch mit erheblichen Nachteilen verbunden. So ist zum einen bei der Zubereitung z.B. eines lyophilisierten oder trocken abgefüllten Präparates vor der Verabreichung immer eine doppelte Handhabung des Wirkstoffes erforderlich, wobei die Trockensubstanz zuerst rekonstituiert und dann verabreicht werden muß. Überdies kann in manchen Fällen für die Auflösung wegen Solubilisierungsproblemen ein längeres Schütteln erforderlich sein.

Weiterhin ist die berufliche Exposition des Pflegepersonals bei der Anfertigung von Lösungen potentiell cancerogener Cytostatika zu beachten, und eine Kontamination des Pflegepersonals sollte deshalb soweit wie möglich vermieden werden. Die Martindale Extra Pharmacopoeia. 28. Auflage. Seite 175, linke Spalte, berichtet diesbezüglich über die nachteiligen Wirkungen von antineoplastischen Wirkstoffen und empfiehlt hierzu, diese Substanzen mit großer Sorgfalt zu handhaben und einen Kontakt mit der Haut und den Augen zu vermeiden. Die Substanzen sollten nicht inhaliert werden. Ferner muß darauf geachtet werden, eine Extravasation zu vermeiden, die zu Schmerzen und Gewebeschädigung führt.

Bei der Herstellung von Lösungen aus Trockensubstanz (Sterilkristallisat, Lyophilisat) kann jedoch eine Einatmung derartiger Wirkstoffpartikel nicht mit Sicherheit ausgeschlossen werden. Im besonderen berichten hierzu Scand. J. Work Environ. Health, Band 10 (2), Seiten 71 bis 74 (1984) sowie Artikel in Chemistry Industry, Ausgabe 4. Juli 1983, Seite 488, und Drug-Topics-Medical-Economics-Co., Ausgabe 7. Februar 1983, Seite 99, über ernste nachteilige Wirkungen, die bei medizinischem Personal beobachtet wurden, welches bei der Verwendung von cytostatischen Mitteln diesen ausgesetzt war.

Aus den vorgenannten Gründen besteht (insbesondere auch in Kliniken) das Bedürfnis nach einer einfach zu handhabenden Oxazaphosphorin-Formulierung zur parenteralen Applikation. Eine solche Zubereitungsform sollte eine Gefährdung des Pflegepersonals soweit wie möglich ausschließen und nicht zuletzt eine kostengünstige Therapie erlauben.

Wäßrige, unmittelbar gebrauchsfertige Lösungen kommen diesem Bedürfnis entgegen, jedoch sind bisher nur flüssige, ethanolische Zubereitungen bekannt. Diese sind ethanolische oder ethanolisch-wäßrige (EP 254 902) bzw. Polyethylen/Propylenglycol (US 4 879 286) enthaltende Lösungen zur Infusion. Diese stellen die bisher einzig verfügbaren flüssigen, jedoch nicht-wäßrigen parenteralen Zubereitungsformen der Oxazaphosphorine dar, insbesondere von Ifosfamid und Cyclophosphamid.

Das in EP 254 902 beschriebene Verfahren zur Herstellung flüssiger Zubereitungsformen der Oxazaphosphorine, insbesondere von Ifosfamid und Cyclophosphamid, beschreibt ethanolische und ethanolisch-wäßrige Lösungen, beispielsweise eine 50%ige (G/G; Gewicht/Gewicht) Lösung von Cyclophosphamid in 96%igem (V/V; Volumen/Volumen) Ethanol, die auch aus Zieckenhuisfarmacie, 1985, Bd. 1, Nr. 2, Seiten 57-58; Martindale, The Extra Pharmacopoeia, 28. Auflage, Seite 199, linke Spalte bekannt ist.

Mit dem in EP 254 902 beschriebenen Verfahren lassen sich mit dem nicht-wäßrigen Lösungsmittel Ethanol als auch mit ethanolisch-wäßrigen Lösungen (80 - 100% (V/V)) hohe Wirkstoffkonzentrationen erzielen (10 - 70% (G/V; Gewicht/Volumen)).

Die ethanolischen Lösungen sind im Vergleich zu Lösungen, die unmittelbar vor der Anwendung aus Trockensubstanz (Sterilkristallisat, Lyophilisat) zubereitet werden, durch eine Reihe von Vorteilen gekennzeichnet:
- Ethanolische und wäßrig-ethanolische Lösungen der Oxazaphosphorine sind stabil und daher für längere Zeit lagerfähig.
- Sie sind weniger der Gefahr einer partikulären oder mikrobiellen Kontamination ausgesetzt.
- Sie lassen eine einfache Entnahme aliquoter Mengen zu.
- Sie sind unmittelbar gebrauchsfertig und tragen damit zur Sicherheit des Plegepersonals bei.
- Sie sind kostengünstiger in der Herstellung.

Der Einsatz solcher hochprozentigen ethanolischen Lösungen ist gegenüber der Trockensubstanz jedoch mit erheblichen Nachteilen verbunden:
- Sie müssen für die parenterale Applikation zuvor mit Infusionsflüssigkeiten verdünnt werden, wobei die Verdünnung so erfolgen muß, daß die maximale Ethanolkonzentration nicht über 10% liegt.
- Sie sind als hochprozentige ethanolische Lösungen als Infusionszusätze, nicht aber für die direkte intravenöse Injektion geeignet.
- Ethanolische Lösungen sind problematisch bei der Verabreichung an Cytostatika-Therapie-bedingt multimorbide Patienten, insbesondere aber auch problematisch bei der Applikation an sogenannte "trockene Alkoholiker".

Die Problematik bei der Applikation hochkonzentrierter ethanolischer und ethanolisch-wäßriger Oxazaphosphorinlösungen konnte auch durch eine Auswahl anderer pharmazeutisch üblicher Hilfsstoffe zur Herstellung direkt injizierbarer stabiler wäßriger Oxazaphosphorinlösungen nicht gelöst werden. EP 254 902 beschreibt hierzu Versuche mit weiteren physiologisch verträglichen Lösungsmitteln, wie Glukofurol, Polyethylenglycol 300, Polyethylenglycol 400, 1,2-Propylenglycol und 1,3-Butylenglycol. Diese Versuche zeigten, daß die Wirkstoffe in diesen Mitteln trotz fehlenden Wassergehaltes ganz erheblich instabiler sind, daß Verfärbungen auftreten und solche Lösungen als lagerfähige pharmazeutische Zubereitungen nicht verwendbar sind.

Das in diesen Versuchen erzielte Ergebnis steht andererseits im Widerspruch zu den un US 4 879 286 geschilderten Vorteilen Polyethylenglycol-/Propylenglycol enthaltender Lösungen zur Infusion.

Eine 10%ige wäßrige Ifosfamidlösung (G/V) zeigt, wie in USP 23-NF, Seite 790 beschrieben, einen pH-Wert von 4,0 bis 7,0; eine 2%ige wäßrige Cyclophosphamidlösung (G/V) zeigt, wie in DAB 10 Kommentar, Grundfg. 1991, 2.Lfg. 1993, C119, Seite 4 beschrieben, ebenfalls einen pH-Wert von 4,0 bis 7,0.

Die Zersetzung dieser Oxazaphosphorine führt über verschiedene Zwischenstufen zu Phosphorsäure und den Syntheseausgangsstoffen, wie beispielsweise für Cyclophosphamid von M. Hirata und Mitarbeitern in Ann. Rept. Shionogi Res. Lab., 1967, Band 17, Seite 103 und in C.A., 1968, Band 69, Seite 21955 beschrieben.

L.A. Trissel und Mitarbeiter beschreiben in Investigational Drug Information. Drug Intell. Clin. Pharm., 1987, Band 12, Seiten 404-405, daß 5%ige und 10%ige (G/V) wäßrige Ifosfamidlösungen, die bei 2 bis 8°C bzw. Raumtemperatur gelagert wurden, nach 17 Wochen bzw. nach 6 Wochen einen bis zu 10%igen Wirkstoffverlust zeigen.

Des weiteren beschreiben D. Brooke und Mitarbeiter in "Chemical stability of cyclophosphamide in parenteral solutions", Am. J. Hosp. Pharm., 1973, Band 30, Seite 134, sowie B. Kirk und Mitarbeiter in "Chemical stability of cyclophosphamide injection", Br. J. Parenter. Ther., 1984, Band 5, Seite 90, daß 2%ige (G/V) wäßrige Cyclophosphamidlösungen, die bei 2 bis 8°C bzw. Raumtemperatur gelagert wurden, nach 17 Wochen bzw. nach 7 Tagen ebenfalls einen bis zu 10%igen Wirkstoffverlust zeigen.

Wegen der Instabilität der Oxazaphosphorine infolge hydrolytischer Zersetzung in wäßriger Lösung, insbesondere von Ifosfamid und Cyclophosphamid, ist die Herstellung direkt injizierbarer lagerstabiler wäßriger Oxazaphosphorinlösungen nach dem Stand der Technik nicht möglich.

EP 538 858 beschreibt ein Verfahren zur Herstellung eines Ifosfamid-Harnstoff Lyophilisates, Formulierungsbestandteile bei der Zubereitung der gefrierzutrocknenden Lösungen sind Wasser in geeigneter pahrmazeutischer Qualität und Harnstoff als hauptsächlicher Hilfsstoff. Als weitere optionale Bestandteile des Ifosfamid-Harnstoff-Lyophilisates werden Mesna (2-Mercaptoethansulfonat) sowie auch in genereller Weise Hilfsstoffe. Puffersubstanzen und Konservierungsmittel genannt. Ein genereller oder bevorzugter pH-Bereich zur Stabilisierung der Wirkstoffe in der gefrierzutrocknenden Lösung und des Lyophilisates wird nicht gezeigt, ebensowenig findet sich irgendein Hinweis auf eine Stabilisierung des Ifosfamids durch Chloridionen.

Aus der WO/9507083 ist ein Verfahren zur Herstellung eines Ifosfamid-Lyophilisats bekannt, bei dem die Lyophilisierung unter Zusatz von Kochsalz erfolgt. Durch den Kochsalz-Zusatz sollen die Eigenschaften des Lyophilisats verbessert werden, insbesondere soll die strukturelle Integrität des nach dem Lyophilisieren erhaltenen Kuchens erhalten bleiben. Kochsalz dient in diesem Verfahren als Gerüststoff für das Lyophilisat, Hinweise darauf, daß Kochsalz zur Stabilisierung des Ifosfamids in wäßrigen Lösungen dienen könnte, finden sich in diesem Dokument nicht.

Eine Aufgabe der vorliegenden Erfindung besteht nun darin, ein Arzneimittel umfassend mindestens einen Wirkstoff aus der Gruppe der Oxazaphosphorine in Form einer lagerstabilen, wäßrigen, unmittelbar gebrauchsfertigen, pyrogenfreien Lösung in einem abgedichteten Behälter bereitzustellen. Eine weitere Aufgabe besteht darin, ein Verfahren zur Herstellung derartiger Lösungen bereitzustellen.

Diese Aufgaben werden durch die Merkmale der unabhängigen Ansprüche gelöst.

Die abhängigen Ansprüche definieren vorteilhafte Ausführungsformen der Erfindung.

Erfindungsgemäß wird eine lagerstabile, gebrauchsfertige wäßrige Arzneilösung bereitgestellt, umfassend einen Wirkstoff aus der Gruppe der Oxaznphosphorine der allgemeinen Formel 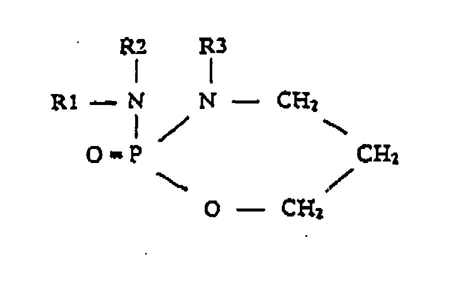 worin R1, R2 und R3 gleich oder verschieden sind und Wasserstoff, Methyl, Ethyl, 2-Chlorethyl oder 2-Methansulfonyloxyethyl bedeuten, mit der Maßgabe, daß R3 nicht Wasserstoff bedeutet, und dabei mindestens zwei dieser Reste 2-Chlorethyl und/oder 2-Methansulfonyloxyethyl sind, sowie eine physiologisch verträgliche, in der wäßrigen Arzneilösung Chloridionen bildende Verbindung.

Als Oxazaphosphorin kommen insbesondere die Wirkstoffe Ifosfamid und Trofosfamid in Frage.

Die Konzentration des Oxazaphosphorins in den erfindungsgemäßen Lösungen beträgt im allgemeinen 0,1 - 50% (G/V), vorzugsweise 0,1 - 30% (G/V) und insbesondere 0,1 - 20% (G/V). Für Ifosfamid sind beispielsweise 0,1 - 50% (G/V) bevorzugt, 0,1 - 30% (G/V) und insbesondere 4 - 20% (G/V) sind am meisten bevorzugt.

Die erfindungsgemäßen Lösungen können auch 2 oder mehr verschiedene Oxazaphosphorin-Wirkstoffe enthalten, und zwar in einer Konzentration als der Summe der Konzentrationen der Wirkstoffe von 0,1 g bis 50 g in 100 ml Lösung, vorzugsweise 0,1 g bis 30 g in 100 ml Lösung und insbesondere 1 g bis 20 g in 100 ml Lösung.

Die erfindungsgemäßen Lösungen werden durch den Zusatz einer Chloridionen bildenden Verbindung stabilisiert. Ohne an eine Theorie gebunden sein zu wollen, wird angenommen, daß der Abbau von Oxazaphosphorinen in den vorbekannten wäßrigen Lösungen auf der Basis der Massenwirkungsbeziehung zwischen Edukt und Produkt erfolgt.

Der Abbau im wäßrigen Medium erfolgt durch intramolekulare Cyclisierung eines Chlorethylaminrestes unter Ausbildung eines Azaridinitunkations mit nachfolgender Abspaltung und Hydratisierung des Chlorid-Anions als zweitem Reaktionsschritt.

Dieser zweite Schritt scheint im wäßrigen System gemäß Masenwirkungsbeziehung entscheidend für die Stabilität des Wirkstoffs zu sein: Ein Chloridionen-Übemhuß (Erhöhung der Pmduktlconxentration) unterdrückt die Abbaureaktion.

Durch Zusatz von Chloridionen im deutlichen Überschuß, beispielsweise 100fach gegenüber der vermuteten Chlorid-Freisetzung kann die Abbaugeschwindiüeit beispielsweise des Ifosfamids in 2-8°C gekühlter wäßriger Lösung deutlich unterdrückt werden.

Die gemäß der Erfindung bevorzugte Chloridionen bildende Verbindung ist Kochsalz, das vorzugsweise in einer Menge von bis zu 1 g/g Wirkstoff in den erfindunssgemäßen Lösungen enthalten ist. Bevorzugt beträgt der Kochsalzgehalt 0,001 g/g Wirkstoff bis 0,25 g/g Wirkstoff, besonders bevorzugt 0,01 g/g Wirkstoff bis 0,15 g/g Wirkstoff.

Erfindungsgemäße, mit NaCl stabilisierte Lösungen zeigen bei einer Lagerung bei 2 - 8°C während eines Jahres eine deutlich verringerte Zersetzung; sie ist nicht höher als 5%.

Die erfindungsgemäße Arzneilösung umfaßt als Lösungsmittel Wasser in pharmazeutisch geeigneter Qualität. Vorzugsweise ist das Wasser in pharmazeutisch geeigneter Qualität das einzige Lösungsmittel. Daneben können die erfindungsgemäßen Lösungen auch einen Alkohol, wie Ethanol, enthalten. Dies kann bei einer Ethattoikonxentration von bis zu 79% (G/V) im Hinblick auf eine weitere Verbesserung der Solubilisierung der Komponenten und eine weitere Verbesserung der Wirkstoffstabilität von Vorteil sein. Da jedoch bei hochkonzentrierten alkoholischen Lösungen, die vorstehend beschriebenen Nachteile bestehen in Bezug auf die Verabreichung als auch in Bezug des Nachteils einer deutlich verminderten Löslichkeit des Harnstoffs sowie der weiteren Hilfsstoffe zur Toniserung als auch zur pH-Einstellung und pH-Stabilisierung der Lösung, sollte eine möglichst geringe Alkoholkonzentration gewählt werden. So kann beispielsweise Ethanol in einer Menge von 0 bis 79 ml in 100 ml Lösung, vorzugsweise von 0 bis 49 ml in 100 ml Lösung und insbesondere von 2 bis 40 ml in 100 ml Lösung eingesetzt werden, wobei 5 bis 30 ml in 100 ml Lösung besonders bevorzugt eingesetzt werden können.

In den Lösungsversuchen zur vorliegenden Erfindung wurde vergleichsweise die Sättigungskonzentration von Ifosfamid in Wasser mit ca. 12% (G/G; Gewicht/Gewicht) und die Sättigungskonzentration von Cyclophosphamid in Wasser mit ca. 6% (G/G) bestimmt.

Durch die optionale Verwendung von Harnstoff und/oder Ethanol als lösungsvermittelnden Hilfsstoff konnte zur Herstellung wäßriger Oxazaphosphorinlösungen erfindungsgemäß eine erhebliche Verbesserung der Löslichkeit, insbesondere von Ifosfamid und Cyclophosphamid, bewirkt werden.

Die Konzentration des Harnstoffes in den erfindungsgemäßen Lösungen beträgt 0,1 - 50% (G/V), vorzugsweise 0,5-30% (G/V) und besonders bevorzugt 1,0 - 20% (G/V).

Beispielsweise vermag Harnstoff in einem Konzentrationsbereich von 0.1 bis 50% (G/G) die Löslichkeit von Ifosfamid in Wasser zu steigern. Insbesondere erhöht sich, wie in Tabelle 1 gezeigt ist, ab einer Harnstoffstartkonzentration von ca. 10% (G/V) die Ifosfamid-Sättigungskonzentration sprunghaft. Bei geeigneter Harnstoffkonzentration kann eine Ifosfamid-Sättigungskonzentration von ca. 50% (G/G) erreicht werden.

**Tabelle 1: Einfluß der Harnstofflconzentration auf die Sättigungskonzentration einer wäßrigen Ifosfamidlösung**

| Harnstoff-Startkonzentration in % (G/V) | Harnstoff-Endkonzentration in % (G/G) | Ifosfamid-Endkonzentration in % (G/G) | pH-Wert |
|---|---|---|---|
| 0 | 0 | 11,8 | 5,2 |
| 1,0 | 0,8 | 12,8 | 5,0 |
| 2,0 | 1,6 | 13,2 | 5,0 |
| 3,0 | 2,4 | 13,9 | 5,0 |
| 4,0 | 3,2 | 14,7 | 5,1 |
| 5,0 | 3,8 | 15,3 | 5,1 |
| 6,0 | 4,4 | 15,9 | 5,1 |
| 7,0 | 4,6 | 16,9 | 5,1 |
| 8,0 | 5,0 | 17,2 | 5,1 |
| 9,0 | 5,1 | 17,3 | 5,2 |
| 10,0 | 6,6 | 37,0 | 5,1 |
| 12,5 | 7,0 | 41,2 | 5,0 |
| 15 | 12,2 | 46,2 | 5,0 |
| 20 | 15,8 | 48,3 | 5,0 |
| 30 | 24,2 | 52,1 | 5,0 |
| 40 | 34,3 | 46,2 | 5,0 |
| 50 | 42,1 | 44,3 | 5,0 |

Der durch die Lösungsversuche in der vorliegenden Erfindung gezeigte löslichkeitsverbessernde Effekt des Harnstoffs, der insbesondere für Ifosfamid ab einer Harnstoffstartkonzentration von ca. 10% (GN), entsprechend ca. 10% (G/G), nahezu sprunghaft einsetzt, war aus dem Stand der Technik nicht bekannt.

Weiterhin vermag Harnstoff in einem Konzentrationsbereich von 0,1 bis 50% (G/G) die Löslichkeit von Cyclophosphamid in Wasser zu verbessern, insbesondere bis zu einer in Tabelle 2 gezeigten Harnstoffstartkonzentration von 50% (G/V). Die Cyclophosphamid-Sättigungskonzentration kann bis auf 17% (G/G) erhöht werden.

**Tabelle 2: Einfluß der Harnstoffkonzentration auf die Sättigungskonzentration einer wäßrigen Cyclophosphamidlösung**

| Harnstoff-Startkonzentration in % (G/V) | Harnstoff-Endkonzentration in % (G/G) | Cyclophosphamid-Endkonzentration in % (G/G) | pH-Wert |
|---|---|---|---|
| 0 | 0 | 6,1 | 3,2 |
| 10 | 9,1 | 8,7 | 3,5 |
| 20 | 17,0 | 11,2 | 3,9 |
| 30 | 24,2 | 13,6 | 4,2 |
| 40 | 30,3 | 17,2 | 4,3 |
| 45 | 37,3 | 17,0 | 4,7 |
| 50 | 45,2 | 15,2 | 4,8 |

Der Änderung des pH-Wertes der erfindungsgemäßen Oxazaphosphorinlösungen als Folge einer geringfügigen Wirkstoffzersetzung kann durch die Verwendung von physiologisch annehmbaren Salzen, physiologisch annehmbaren Alkalisierungs- und/oder Ansäuerungsmitteln begegnet werden. So können beispielsweise saure oder basische Alkalisalze der Phosphorsäure sowie Salzsäure, Phosphorsäure oder Natronlauge zur Einstellung und Stabilisierung des pH-Wertes der Lösung eingesetzt werden.

Ebenso wird durch die Verwendung von physiologisch annehmbaren Salzen, physiologisch annehmbaren Alkalisierungs- und/oder Ansäuerungsmitteln der pH-Wert im Bereich des Stabilitätsoptimums von Harnstoff, soweit enthalten, gehalten, wie von W. Süß in Pharmazie, 1965, Band 20, Seite 34-37 beschrieben.

Die Einstellung des pH-Wertes der erfindungsgemäßen Lösungen erfolgt hierbei auf pH-Werte innerhalb des pH-Bereiches von 4,5 bis 10,0. Bevorzugte pH-Bereiche sind 5,0 bis 9,5, 6,5 bis 9,0, 7,5 bis 9,5, 7,5 bis 9,0 und 7,5 bis 8,0. Ein weiterer bevorzugter pH-Bereich ist 7.5 bis 8.0. Geeignet ist auch ein pH-Bereich von 7,0 bis 8,5. Ein besonders bevorzugter pH-Bereich ist ein pH von 7.4 bis 8,0. Ein weiterer besonders bevorzugter pH-Bereich ist 7.3 bis 8,5.

Die erfindungsgemäßen Lösungen können zur Einstellung und Stabilisierung des pH-Wertes der Lösungen physiologisch annehmbare Salze, physiologisch annehmbare Alkalisierungsmittel und/oder physiologisch annehmbare Ansäuerungsmittel enthalten. Es können anorganische und/oder organische Säuren und/oder anorganische Salze und/oder organische Salze sowie anorganische Basen und/oder organische Basen eingesetzt werden. Es können auch die sauren, neutralen und alkalischen Salze und ihre Hydrate eingesetzt werden.

Beispiele für anorganische Säuren (Mineralsäuren) sind Salzsäure, Bromwasserstoffsäure, Schwefelsäure, Kohlensäure, Salpetersäure und Phosphorsäure. Beispiele für anorganische Basen sind Ammoniumhydroxid, Alkalihydroxide, wie Kaliumhydroxid und Natriumhydroxid, sowie Erdalkalihydroxide, wie Calciumhydroxid und Magnesiumhydroxid.

Beispiele für organische Säuren sind Milchsäure, Essigsäure, Bernsteinsäure, Oxalsäure, Weinsäure, Ascorbinsäure, Äpfelsäure, Zitronensäure, Fumarsäure, Glutaminsäure, Benzoesäure, Methansulfonsäure und Ethansulfonsäure. Beispiele für organische Basen sind Tromethamin (Trometamol) und N-Methylglucamin (Meglumin) sowie die korrespondierenden Basen der Bernsteinsäure, Oxalsäure, Weinsäure, Ascorbinsäure, Äpfelsäure, Zitronensäure, Fumarsäure, Glutaminsäure, Benzoesäure, Methansulfonsäure und Ethansulfonsäure in der Form ihrer Alkali- und Erdalkalisalze.

In den erfindungsgemäßen Lösungen können auch Salze organischer Basen, wie Mineralsäuresalze von Tromethamin (Trometamol) und N-Methylglucamin (Meglumin), vorzugsweise Trometamol-Hydrochlorid und Meglumin-Hydrochlorid, eingesetzt werden.

Als bevorzugte Komponenten zur Einstellung und Stabilisierung des pH-Wertes der Lösung werden Phosphorsäure und Salze der Phosphorsäure sowie deren Hydrate verwendet, wobei Dinatriumhydrogenphosphat-Dihydrat und Natriumdihydrogenphosphat-Dihydrat besonders bevorzugt werden. Weitere bevorzugte Pufferkomponenten sind Natriumlactat, Natriumacetat, Natriumacetat-Trihydrat und Trinatriumcitrat-Trihydrat.

Besonders bevorzugte Pufferkomponenten, Alkalisierungsmittel bzw. Ansäuerungsmittel sind Salze der Phosphorsäure, sowie Phosphorsäure, Salzsäure und Natronlauge.

Die erfindungsgemäßen Lösungen können, wie bereits erwähnt, 0,1 bis 50% (G/V) Harnstoff enthalten. Wie von W. Horsch und B. Wolf in: Die Pharmazie, Oktober 1985, Heft 10, 40. Jahrgang, Seite 666, linke Spalte unten und von J.G. Schmidt und Mitarbeitern in Sterilisation, Desinfektion und Entwesung, Leipzig, 1968 beschrieben, sind isosmolare Harnstofflösungen hypoton; hochkonzentrierte hyperosmotische Harnstofflösungen weisen eine nur geringfügige Tonizität auf. Diese Eigenschaft des Harnstoffs im Hinblick auf die Tonizität wäßriger Lösungen, insbesondere der wäßrigen Lösungen der vorliegenden Erfindung, wird durch die erfindungsgemäße Verwendung von physiologisch annehmbaren Tonisierungsmitteln, beispielsweise von physiologisch annehmbaren Salzen und/oder Kohlenhydraten, berücksichtigt. Die Tonizität der erfindungsgemäßen Lösungen kann auch mit den erfindungsgemäßen Salzen, Alkalisierungs- und Ansäuerungsmitteln in einem weiten Variationsbereich eingestellt werden.

Im Vergleich zu ethanolischen Lösungen (EP 254 902) ermöglicht das erfindungsgemäße wäßrige Lösungsmittel der vorliegenden Erfindung die Einstellung der Tonizität der erfindungsgemäßen Oxazaphosphorinlösungen sowohl als isotone als auch als hypertone Lösungen mit physioloigsch annehmbaren, ionischen und/oder nicht-ionischen Tonisierungsmitteln. Diese Tonisierungsmittel sind in ethanolischen Lösungen nicht oder nicht ausreichend zur Einstellung der Tonizität, d.h. zur Herstellung sowohl isotoner als auch hypertoner Lösungen, löslich.

Somit wird es erst durch das wäßrige Lösungssystem der vorliegenden Erfindung ermöglicht, (hyperosmolar-)hypertone als auch (hyperosmolar-)isotone, unmittelbar gebrauchsfertige Oxazaphosphorinlösungen zur Verfügung zu stellen, die beispielsweise zur Infusion direkt aus der Injektionsflasche in die Infusionslösung überführt werden können.

Durch das wäßrige Lösungssystem der vorliegenden Erfindung wird es überdies insbesondere ermöglicht, (hyperosmolar-)isotone, unmittelbar gebrauchsfertige Oxazaphosphorinlösungen zur intravenösen Injektion als auch vernebelt zur Inhalation für Mensch und Tier bereitzustellen zur Behandlung verschiedener Tunorerkrankungen und anderer maligner Erkrankungen sowie zur Behandlung von Autoimmunerkrankungen, beispielsweise Lupus erythematodes, nephrotisches Syndrom, Sklerodermie, rheumatoide Arthritis, Arthrophathia psoriatica, autoimmunhämolytische Anämie und Myasthenia gravis sowie zur Behandlung im Rahmen der Immunsuppression nach Organ- und Knochenmarktransplantation.

Die bevorzugten erfindungsgemäßen Lösungen enthalten physiologisch annehmbare Hilfsstoffe zur Einstellung der Tonizität, wie physiologisch annehmbare Salze und/oder physiologisch annehmbare Kohlenhydrate. Dabei kann die Tonizität gemäß einer besonders bevorzugten Ausführungsform allein durch die zur Stabilisierung des Wirkstoffes verwendete Chloridionen bildende Verbindung, bevorzugt Kochsalz, erfolgen. Zusätzlich kann als physiologisch annehmbares Tonizitätseinstellungsmittel mindestens ein Salz enthalten sein, das aus der Gruppe der anorganischen Salze und/oder ihrer Hydrate, beispielsweise aus der Gruppe der Salze und ihrer Hydrate der anorganischen Säuren (Mineralsäuren), vorzugsweise der Salze der Salzsäure.

Schwefelsäure, Salpetersäure, Phosphorsäure oder Kohlensäure, ausgewählt ist.

Das physiologisch annehmbare Tonizitätseinstellungsmittel kann auch mindestens ein Salz sein, das aus der Gruppe der organischen Salze und/oder ihrer Hydrate, beispielsweise aus der Gruppe der Salze und ihrer Hydrate der organischen Säuren, vorzugsweise der Salze der Milchsäure, Essigsäure, Bernsteinsäure, Oxalsäure, Weinsäure, Ascorbinsäure, Äpfelsäure, Zitronensäure, Fumarsäure, Glutaminsäure, Benzoesäure, Methansulfonsäure und Ethansulfonsäure, ausgewählt ist, wobei ein besonders bevorzugtes Salz das Natriumlactat ist.

Ein weiteres physiologisch annehmbares Tonizitätseinstellungsmittel kann auch Kreatinin sein.

Ferner kann das physiologisch annehmbare Tonizitätseinstellungsmittel mindestens ein Kohlenhydrat sein, das aus der Gruppe der 3-9C-Polyhydroxyalkohole ausgewählt ist.

Weiterhin kann es sich um ein Kohlenhydrat handeln, das aus der Gruppe der 5-10C-Monosaccharide, beispielsweise der Pentite, Pentosen, Hexite und Hexosen, ausgewählt ist, wobei es sich vorzugsweise um Dextrose, Sorbit oder Mannit handelt und wobei Sorbit oder Mannit besonders bevorzugt werden. Es kann sich auch um mindestens ein Kohlenhydrat handeln, das aus der Gruppe der Disaccharide ausgewählt ist, beispielsweise Maltose, Saccharose, Lactose, Maltobiose und Maltotriose. Vorzugsweise handelt es sich um Maltose, Saccharose oder Lactose. Das physiologisch annehmbare Tonizitätseinstellungsmittel kann auch mindestens ein Kohlenhydrat sein, das aus der Gruppe der Polysaccharide ausgewählt ist, wie beispielsweise Stärke und Dextrane.

Weitere als physiologisch annehmbare Tonizitätseinstellungsmittel geeignete Kohlenhydrate sind aus der Gruppe der Cyclodextrine, beispielsweise der α-, β- und γ-Cyclodextrine und ihrer ethoxyalkylierten Derivate ausgewählt. Bevorzugt werden α-, β- und γ-Cyclodextrine, wobei α-Cyclodextrine besonders bevorzugt werden.

Geeignete Komponenten zur Einstellung der Tonizität umfassen also beispielsweise Glycerin (Glycerol), Threit, Trehalose, Erythrit, Pentaerythrit, Erythrose, Arabit, Arabinose, Adonit, Xylit, Xylulose, Sorbit, Sorbose, Mannit, Mannose, Dulcit, Dextrose (Glucose), Fructose, Maltose, Saccharose, Lactose, Maltobiose, Maltotriose, Stärke, Hydroxyethylstärke (Dextrane), α-, β- und γ-Cyclodextrine sowie ihre alkoxyethylierten Derivate, vorzugsweise Threit, Erythit, Arabit, Adonit, Xylit, Sorbit, Mannit, Dulcit, Dextrose (Glucose), Fructose, Maltose, Saccharose sowie Lactose und insbesondere Sorbit, Mannit, Dextrose (Glucose), Fructose sowie Lactose, wobei Hexite, wie Sorbit und/oder Mannit, besonders bevorzugt sind.

Die am meisten bevorzugten physiologisch unbedenklichen Tonizitätseinstellungsmittel sind neben Natriumchlorid, Hexite, wie Mannit, und/oder Sorbit.

Die erfindunsgemäße Arzneilösung kann als weiteren Bestandteil Natrium-2-mercaptoethansulfonat (Mesna) enthalten, das als Uroprotectivum wirkt. Dieses Entgiftungsmittel wird beispielsweise in einer Menge von 0,1 bis 1 Gew.-Teil pro Gewichtsteil des Oxazaphosphorins eingesetzt. Der Einsatz anderer Uroprotektiva, allein oder in Kombination mit Mesna, ist erfindungsgemäß ebenfalls möglich.

Die vorliegende Erfindung betrifft weiterhin ein Verfahren zur Herstellung lagerstabiler, wäßriger, steriler, pyrogenfreier, in Behältern verschlossener, unmittelbar gebrauchsfertiger Oxazaphosphorinlösungen, die durch eine Chloridionen bildende Verbindung stabilisiert sind.

Bei dem Verfahren können die Komponenten in beliebiger Reihenfolge in Wasser gelöst werden. Zur Verbesserung der Solubilisierung der Komponenten können gegebenenfalls auch Ethanol und/oder Harnstoff eingesetzt werden. Die erfindungsgemäßen Lösungen können also hergestellt werden, indem man Oxazaphosphorine der vorstehend angegebenen Formel und die stabilisierende Verbindung zweckmäßigerweise bei einer Temperatur zwischen 5 und 40°C, in Wasser pharmazeutisch annehmbarer Qualität, beziehungsweise zur Beschleunigung der Auflösung gegebenenfalls in harnstoffhaltigem Wasser mit einer Harnstoffkonzentration von 0,1 - 50% (G/V), sowie gegebenenfalls physiologisch annehmbare Hilfsstoffe auflöst, wobei auf 100 ml Lösungsmittel 0,1 bis 50 g, vorzugsweise 0,1 bis 30 g, insbesondere 1 bis 20 g Oxazaphosphorin verwendet werden.

Bei dem erfindungsgemäßen Verfahren können die Oxazaphosphorine zum Auflösen also in Wasser geeigneter Qualität gegeben werden, oder sie können in eine wäßrige Lösung gegeben werden, die bereits einen Teil oder die Gesamtmenge der Hilfsstoffe enthält.

Zum Auflösen werden die Oxazaphosphorine vorzugsweise in kristalliner Form (zum Beispiel feinkristalliner Form) eingesetzt. Selbstverständlich können für die Auflösung aber auch amorphe, halbfeste oder ölige Formen der Oxazaphosphorine verwendet werden. Für die Herstellung der Lösungen von Oxazaphosphorinen der vorstehend angegebenen Formel und den genannten Hilfsstoffen zur Stabilisierung des Wirkstoffes, zur Einstellung der Tonizität und des pH-Wertes der Lösung als auch zur Stabilisierung des pH-Wertes der Lösung können diese auch als Einzelkomponenten oder auch als ihre Gemische in der Form ihrer Lyophilisate aus diesen mit Wasser als auch mit einem anderen physiologisch annehmbaren, beispielsweise alkoholisch-wäßrigen Lösungsmittelgemisch rekonstituiert eingesetzt werden. Das Auflösen kann gegebenenfalls unter Rühren oder sonstiger Bewegung des Lösungsmittels erfolgen.

Die Einstellung des pH-Wertes der erfindungsgemäßen Lösungen kann auf pH-Werte innerhalb des pH-Bereiches von 4,5 bis 10,0, vorzugsweise von 6,5 bis 9,0, insbesondere von 7,0 bis 8,5 erfolgen. Ein besonders bevorzugter pH-Bereich ist ein pH von 7,4 bis 8,0.

Die erfindungsgemäße Lösung, wie sie vorstehend hergestellt wurde, läßt man vor dem Einfüllen in einen Behälter durch einen Sterilisierungsfilter laufen (Sterilfiltration). Anschließend wird der Behälter verschlossen. Die Lösung ist dann gebrauchsfertig als sterile, pyrogenfreie, wäßrige pharmazeutische Darreichungsform für die Anwendung an Mensch und Tier für die intravenöse Injektion, intravenöse Infusion und vernebelt zur Inhalation.

Die erfindungsgemäßen Lösungen können für die parenterale Applikation und Inhalation auf einfache Weise mit Wasser, Ringer-Lösung, 5%iger Glucoselösung oder ähnlichen Infusionslösungen verdünnt werden. Sie können beispielsweise direkt aus der Injektionsflasche in die Infusionslösung überführt werden. Sie können aber auch als isotone Lösungen intravenös injiziert oder vernebelt inhaliert werden. In der Therapie von Tumorerkrankungen hierfür besonders bevorzugte Konzentrationen sind beispielsweise 4%ige Ifosfamidlösungen für die intravenöse Applikation.

Die erfindungsgemäßen Lösungen weisen überraschenderweise eine ausgezeichnete Stabilität auf und sind daher für längere Zeit lagerfähig. Insbesondere zeichnen sich die erfindungsgemäßen Lösungen gegenüber bekannten festen und flüssigen Zubereitungen aus durch verbesserte Löslichkeit und verbesserte Stabilität der Oxazaphosphorine in wäßriger Lösung bei einer Lagertemperatur von 2 bis 8°C. Einen weiteren Vorteil der Lösungen stellt der weitgehende Ausschluß von Gefährdungsrisiken des Pflegepersonals als auch des Patienten durch die erfindungsgemäße Zurverfügungstellung von unmittelbar gebrauchsfertigen Lösungen für die parenterale Applikation dar.

Die erfindungsgemäßen wäßrigen Lösungen der Oxazaphosphorine, insbesondere von Ifosfamid, zeichnen sich im Vergleich mit Lösungen, die unmittelbar vor der Anwendung aus Sterilkristallisaten oder Lyophilisaten zubereitet werden, als auch gegenüber ethanolischen bzw. ethanolisch-wäßrigen Lösungen, durch eine Reihe von Vorteilen aus:
- Sie sind bei einer pharmazeutisch annehmbaren Lagertemperatur von 2 - 8°C stabil und sind daher für längere Zeit lagerfähig.
- Sie enthalten mit dem bevorzugten Stabilisator Kochsalz eine physiologische Substanz, die im Gegensatz zu Ethanol keiner weiteren Metabolisierung unterliegt.
- Sie können mit Infusionslösungen verdünnt als auch unverdünnt auch mit hohen Konzentrationen an Harnstoff, beispielsweise als isotone Lösung mit 30% Harnstoff, intravenös appliziert werden (ethanolische Lösungen müssen für die parenterale Applikation auf maximale Ethanolkonzentrationen unter 10% verdünnt werden).
- Sie sind weniger der Gefahr einer partikulären oder mikrobiellen Kontamination ausgesetzt (da zum Beispiel unmittelbar vor der Abfüllung filtriert werden kann).
- Sie lassen eine einfache Entnahme aliquoter Mengen zu, wobei der Wirkstoffgehalt stets einheitlich ist (bei einer Trockenabfüllung liegen stets Schwankungen hinsichtlich des Wirkstoffs vor).
- Sie machen den Lösungsvorgang bei der Anwendung überflüssig, sind unmittelbar gebrauchsfertig und ermöglichen damit eine einfachere Handhabung.
- Sie ermöglichen als unmittelbar gebrauchsfertige Lösung eine Verringerung der Gefährdungsrisiken und tragen damit zur Sicherheit des Pflegepersonals und zur Sicherheit des Chemotherapie-bedingt immunsupprimierten Patienten bei.

Die Einstellung der Tonizität der erfindungsgemäßen Oxazaphosphorinlösungen bietet Vorteile bei der Handhabung und Anwendung. Durch die Einstellung der Tonizität sind die erfindungsgemäßen wäßrigen Oxazaphosphorinlösungen sowohl als isosmolare als auch im besonderen als hochkonzentrierte, hyperosmolare wäßrige Lösungen im Gegensatz zur Ethanol (EP 254 902) auch unverdünnt intravenös als isotone Lösungen parenteral applizierbar.

Mit der vorliegenden Erfindung ist es erstmals möglich, stabile wäßrige und unmittelbar gebrauchsfertige Oxazaphosphorinlösungen mit einem weiten Wirkstoffkonzentrationsbereich sowohl als isotone Lösung zur Injektion und Infusion oder auch als hypertones Lösungskonzentrat zur Infusion zur Verfügung zu stellen. In der Therapie von Tumorerkrankungen hierfür besonders bevorzugte Konzentrationen sind beispielsweise 4%ige Ifosfamidlösungen für die intravenöse Applikation (Injektion, Infusion).

Schließlich sind die erfindungsgemäßen Lösungen kostengünstiger in der Herstellung als andere Darreichungsformen und minimieren in der Darreichungsform eines volumenreduzierten Lösungskonzentrates die Kosten für die Kliniksondermüllentsorgung.

Die folgenden Beispiele verdeutlichen die Erfindung.

### Beispiel 1: Herstellung einer 4%igen Ifosfamidlösung

A) Ifosfamid 40 mg/ml Solution 1000 mg
B) Ifosfamid 40 mg/ml Solution 2000 mg

| Zusammensetzung: | ad 1 l | A+B) für je 500 Vial | A) für 1 Vial | B) für 1 Vial |
|---|---|---|---|---|
| Ifosfamid | 40,00 g | 1500,00 g | 1,000 g | 2,000 g |
| Natriumchlorid | 9,0 g | 337,5 g | 0,225 g | 0,450 g |
| Wasser für Inj.-Zwecke | ad 1 l | ad 37,5 l | 25,0 ml | 50,0 ml |

In einem geeigneten Behälter werden etwa 90% der insgesamt erforderlichen Menge Wasser für Injektionszwecke vorgelegt und unter Rühren der Stabilisator Natriumchlorid zugegeben. Der Wirkstoff Ifosfamid wird zugegeben und unter Rühren aufgelöst. Der Ansatz wird mit Wasser für Injektionszwecke auf das Ansatzendgewicht aufgefüllt. Der Ansatz wird durch Hindurchperlen von Stickstoff entlüftet. Die Lösung wird unter Stickstoffdruck durch eine mikroporöse 0,2 µm-Membran sterilfiltriert. Zur Herstellung der Injektionsflaschen mit 25 ml bzw. 50 ml Lösung wird unter aseptischen Bedingungen in bekannter Weise in farblose 25-ml-Injektionsflaschen bzw. 50-ml-Injektionsflaschen dosiert, und diese werden dann mit Stopfen aus Brombutylkautschuk verschlossen und mit Aluminiumkappen abgedichtet.

### Beispiel 2: Herstellung einer 4%igen Ifosfamidlösung

A) Ifosfamid 40 mg/ml Solution 1000 mg
B) Ifosfamid 40 mg/ml Solution 2000 mg

| Zusammensetzung: | ad 1 l | A+B) für je 500 Vial | A) für 1 Vial | B) für 1 Vial |
|---|---|---|---|---|
| Ifosfamid | 40,00 g | 1500,00 g | 1,000 g | 2,000 g |
| Harnstoff | 30,00 g | 1125,00 g | 0,750 g | 1,500 g |
| Dinatriumhydrogen-phosphat-Dihydrat | 6,25 g | 234,38 g | 0,156 g | 0,312 g |
| Natriumchlorid | 0,80 g | 30,00 g | 0,020 g | 0,040 g |
| Salzsäure, 1 N | q.s. | q.s. | | |
| Wasser für Inj.-Zwecke | ad 1 l | ad 37,5 l | 25,0 ml | 50,0 ml |

In einem geeigneten Behälter werden etwa 90% der insgesamt erforderlichen Menge Wasser für Injektionszwecke vorgelegt und unter Rühren der Stabilisator Natriumchlorid und der Puffer Dinatriumhydrogenphosphat-Dihydrat zugegeben. Nach Auflösen dieser Substanzen wird der pH-Wert der Lösung mit Salzsäure eingestellt auf pH 7,8. Anschließend wird der Hilfsstoff Harnstoff unter Rühren aufgelöst und der pH-Wert erneut eingestellt auf pH 7,8. Der Wirkstoff Ifosfamid wird zugegeben und unter Rühren aufgelöst. Der Ansatz wird mit Wasser für Injektionszwecke auf das Ansatzendgewicht aufgefüllt und der pH-Wert erneut eingestellt auf pH 7,8. Der Ansatz wird durch Hindurchperlen von Stickstoff entlüftet. Die Lösung wird unter Stickstoffdruck durch eine mikroporöse 0,2 µm-Membran sterilfiltriert. Zur Herstellung der Injektionsflaschen mit 25 ml bzw. 50 ml Lösung wird unter aseptischen Bedingungen in bekannter Weise in farblose 25-ml-Injektionsflaschen bzw. 50-ml-Injektionsflaschen dosiert, und diese werden dann mit Stopfen aus Brombutylkautschuk verschlossen und mit Aluminiumkappen abgedichtet.

### Beispiel 3: Herstellung einer 20%igen Ifosfamidlösung

A) Ifosfamid 200 mg/ml Solution 1000 mg,
B) Ifosfamid 200 mg/ml Solution 2000 mg

| Zusammensetzung: | ad 11 | A+B) für je 500 Vial | A) für 1 Vial | B) für 1 Vial |
|---|---|---|---|---|
| Ifosfamid | 200,00 g | 500,00 g | 1,000 g | 2,000 g |
| Harnstoff | 150,00 g | 375,00 g | 0,750 g | 1,500 g |
| Dinatriumhydrogen-phosphat-Dihydrat | 15,00 g | 37,50 g | 0,075 g | 0,150 g |
| Natriumchlorid | 4,00 g | 10,00 g | 0,020 g | 0,040 g |
| Salzsäure, 1 N | q.s. | q.s. | | |
| Wasser für | | | | |
| Inj.-Zwecke | ad 1 l | ad 7,5 l | 5,0 ml | 10,0 ml |

In einem geeigneten Behälter werden etwa 90% der insgesamt erforderlichen Menge Wasser für Injektionszwecke vorgelegt und unter Rühren der Stabilisator Natriumchlorid und der Puffer Dinatriumhydrogenphosphat-Dihydrat zugegeben. Nach Auflösen dieser Hilfsstoffe wird der pH-Wert der Lösung mit Salzsäure eingestellt auf pH 7,8. Anschließend wird der Hilfsstoff Harnstoff unter Rühren aufgelöst und der pH-Wert erneut eingestellt auf pH 7,8. Der Wirkstoff Ifosfamid wird zugegeben und unter Rühren aufgelöst. Der Ansatz wird mit Wasser für Injektionszwecke auf das Ansatzendgewicht aufgefüllt und der pH-Wert erneut eingestellt auf pH 7,8. Der Ansatz wird durch Hindurchperlen von Stickstoff entlüftet. Die Lösung wird unter Stickstoffdruck durch eine mikroporöse 0,2 µm-Membran sterilfiltriert. Zur Herstellung der Injektionsflaschen mit 5 ml bzw. 10 ml Lösung wird unter aseptischen Bedingungen in bekannter Weise in farblose 6-ml-Injektionsflaschen bzw. 10-ml-Injektionsflaschen dosiert, und diese werden dann mit Stopfen aus Brombutylkautschuk verschlossen und mit Aluminiumkappen abgedichtet.

### Beispiel 4

Entsprechend den in den vorgenannten Beispiel beschriebenen Verfahren wurden rein wäßrige Ifosfamidlösungen folgender Zusammensetzungen hergestellt (Gehaltsangaben jeweils in mg/ml Lösung):

| Ifosfamid | Harnstoff | Natriumchlorid | Dinatriumhydrogen-phosphat-Dihydrat | pH-Wert |
|---|---|---|---|---|
| 20 | 10 | 5 | 2 | 7,2-7,4 |
| 40 | 20 | 4 | 0 | 5,5-6,0 |
| 80 | 50 | 2 | 0 | 5,5-6,0 |
| 100 | 50 | 5 | 1 | 6,0-6,5 |
| 100 | 100 | 10 | 2 | 5,5-6,0 |
| 100 | 100 | 5 | 0 | 5,5-6,0 |
| 100 | 100 | 12,5 | 0 | 5,5-6,0 |
| 100 | 120 | 10 | 5 | 7,0-7,4 |
| 200 | 150 | 10 | 0 | 5,5-6,0 |
| 200 | 200 | 5 | 5 | 7,4-7,8 |
| 250 | 200 | 5 | 2 | 7,6-7,8 |

### Beispiel 5

Entsprechend den in den vorgenannten Beispielen beschriebenen Verfahren wurden ethanolisch-wäßrige Lösungen folgender Zusammensetzungen hergestellt (Gehaltsangaben für die Feststoffe in mg/ml Lösung, für Ethanol in Volumenprozent):

| Ifosfamid | Ethanol | Harnstoff | Natriumchlorid | Puffersalz | pH-Wert |
|---|---|---|---|---|---|
| 100 | 10 Vol.% | 100 | 10 | 5 | 7,0 |
| 100 | 10 Vol.% | 0 | 10 | 0 | 5,0-6,0 |
| 200 | 10 Vol.% | 150 | 10 | 5 | 6,5-7,0 |
| 100 | 20 Vol.% | 100 | 10 | 5 | 7,6 |
| 100 | 20 Vol.% | 0 | 10 | 0 | 5,0-6,0 |
| 100 | 20 Vol.% | 0 | 10 | 5 | 6,5-7,5 |
| 200 | 20 Vol.% | 150 | 10 | 5 | 7,5-7,8 |
| 200 | 50 Vol.% | 0 | 20 | 10 | 7,0-7,4 |
| 250 | 50 Vol.% | 100 | 10 | 5 | 6.5-7.0 |
| 200 | 75 Vol.% | 0 | 10 | 5 | 6,0-6,5 |
| 400 | 75 Vol.% | 100 | 5 | 0 | 5,5-6,0 |

### Beispiele 6 bis 9

In den folgenden Beispielen werden die Stabilitätsdaten von unstabilisierten, rein wäßrigen Ifosfamidlösungen und erfindungsgemäßen, durch Zusatz von Natriumchlorid stabilisierten Ifosfamidlösungen ermittelt und gegenübergestellt.

### Beispiel 6 (Vergleichsbeispiel)

Es wurden rein wäßrige Ifosfamidlösungen einer Konzentration von 40 mg Ifosfamid pro ml Lösung hergestellt und deren Ifosfamidgehalte bei Lagerung bei 2-8°C, 15°C, 25°C und 40°C über einen Zeitraum von 15 Monaten mittels HPLC ermittelt.

Die erhaltenen Werte sind in Tabelle 3 zusammengefaßt.

**Tabelle 3: Stabilitätsdaten einer unstabilisierten, rein wäßrigen Ifosfamidlösung. Die Gehalte sind in % bezogen auf einen Anfangsgehalt von 100% angegeben.**

| Zeit | Rel.Gehalt nach Lagerung 2-8°C | Rel.Gehalt nach Lagerung 15°C | Rel.Gehalt nach Lagerung 25°C | Rel.Gehalt nach Lagerung bei 40°C |
|---|---|---|---|---|
| 0 | 40,54 | 40,54 | 40,54 | 40,54 |
| | 100,0% | 100,0% | 100,0% | 100,0% |
| | pH 5,8 | pH 5,8 | pH 5,8 | pH 5,8 |
| 6 Wochen | - | 38,63 | 35,67 | 13,42 |
| | | 95,3% | 88,0% | 33,1% |
| | | pH 5,6 | pH 5,5 | pH 4,7 |
| 3 Monate | 39,04 | 37,58 | 32,51 | 1,01 |
| | 96,3% | 92,7% | 80,2% | 2,5% |
| | pH 5,6 | pH 5,5 | pH 5,3 | pH 4,0 |
| 5 Monate | 36,61 | 33,73 | 29,27 | 0,45 |
| | 90,3% | 83,2% | 72,2% | 0,11% |
| | pH 5,4 | pH 5,3 | pH 5,1 | pH 3,6 |
| 7 Monate | 34,90 | - | - | - |
| | 86,1% | | | |
| | pH 5,3 | | | |
| 11 Monate | 33,73 | 30,28 | 25,70 | - |
| | 83,2% | 74,7% | 63,4% | |
| | pH 5,2 | pH 5,1 | pH 4,8 | |
| 15 Monate | 31,70 | 30,28 | 25,70 | - |
| | 78,2% | 74,7% | 63,4% | |
| | pH 5,2 | pH 5,1 | pH 4,8 | |

Es zeigt sich, daß bei einer Lagerung bei 2-8°C bereits nach 5 Monaten ca. 10% des Ifosfamids abgebaut worden sind, was aus pharmazeutischer Sicht nicht akzeptabel ist.

### Beispiel 7

Die zuvor beschriebene Stabilitätsuntersuchung wurde mit einer erfindungsgemäßen Ifosfamidlösung gemäß Beispiel 1 wiederholt. Die erhaltenen Ergebnisse sind in Tabelle 4 zusammengefaßt.

**Tabelle 4: Stabilitätsdaten einer erfindungsgemäßen, durch Zusatz von NaCl stabilisierten Ifosfamidlösung.**

| Zeit | Rel.Gehalt nach Lagerung 2-8°C | Rel.Gehalt nach Lagerung 15°C | Rel.Gehalt nach Lagerung 25°C | Rel.Gehalt nach Lagerung bei 40°C |
|---|---|---|---|---|
| 0 | 40,14 | 40,14 | 40,14 | 40,14 |
| | 100,0% | 100,0% | 100,0% | 100,0% |
| | pH 5,7 | pH 5,7 | pH 5,7 | pH 5,7 |
| 6 Wochen | - | 39,66 | 36,53 | 18,75 |
| | | 98,8% | 91,0% | 46,7% |
| | | pH 5,7 | pH 5,5 | pH 4,8 |
| 3 Monate | 40,14 | 39,42 | 33,76 | 3,81 |
| | 100,0% | 98,2% | 84,1% | 9,5% |
| | pH 5,7 | pH 5,7 | pH 5,4 | pH 4,4 |
| 5 Monate | 40,66 | 38,57 | 31,39 | 0,43 |
| | 101,3% | 96,1% | 78,2% | 0,1% |
| | pH 5,7 | pH 5,6 | pH 5,3 | pH 3,6 |
| 7 Monate | 40,87 | - | - | - |
| | 101,8% | | | |
| | pH 5,8 | | | |
| 11 Monate | 39,30 | 36,49 | 28,14 | - |
| | 97,9% | 90,9% | 70,1% | |
| | pH 5,7 | pH 5,2 | pH 5,1 | |
| 15 Monate | 39,62 | - | - | |
| | 98,7% | | | |
| | pH 5,6 | | | |

Es zeigt sich, daß nach 15 monatiger Lagerung bei 2-8°C keine nennenswerte Zersetzung des Ifosfamids auftritt. Ferner zeigt sich, daß der pH-Wert bei diesen Bedingungen auch ohne Verwendung eines Puffersystems stabil bleibt.

### Beispiel 8

Die zuvor beschriebene Stabilitätsuntersuchung wurde mit einer erfindungsgemäßen Lösung gemäß Beispiel 2 wiederholt. Die erhaltenen Ergebnisse sind in Tabelle 5 zusammengefaßt.

**Tabelle 5: Stabilitätsdaten einer erfindungsgemäßen, stabilisierten, gepufferten Ifosfamidlösung, die als Hilfsmittel Harnstoff umfaßt.**

| Zeit | Rel.Gehalt nach Lagerung 2-8°C | Rel.Gehalt nach Lagerung 15°C | Rel.Gehalt nach Lagerung 25°C | Rel.Gehalt nach Lagerung bei 40°C |
|---|---|---|---|---|
| 0 | 40,90 | - | 40,90 | 40,90 |
| | 100,0% | | 100,0% | 100,0% |
| | pH 7,8 | | pH 7,8 | pH 7,8 |
| 2 Monate | 40,41 | - | 40,46 | 11,99 |
| | 98,80% | | 98,92% | 29,32% |
| | pH 7,6 | | - | - |
| 4 Monate | 39,78 | - | 35,35 | 1,23 |
| | 97,26% | | 86,43% | 3,01% |
| | pH 7,6 | | pH 6,3 | pH 4,8 |
| 6 Monate | 39,40 | - | 33,63 | |
| | 96,33% | | 82,22% | |
| | pH 7,6 | | pH 6,2 | |
| 9 Monate | 39,51 | - | 30,00 | - |
| | 96,60% | | 73,35% | |
| | pH 7,5 | | pH 5,7 | |
| 12 Monate | 39,21 | - | - | - |
| | 95,87% | | | |
| | pH 7,6 | | | |
| 15 Monate | 40,32 | - | - | - |
| | 98,58% | | | |
| | pH 7,6 | | | |

Auch hier zeigt sich die hervorragende Lagerstabilität der erfindungsgemäßen Lösungen. Im Rahmen der Meßgenauigkeit tritt nach einer Lagerung bei 2-8°C nach 15 Monaten praktisch keine Zersetzung des Wirkstoffes auf.

### Beispiel 9

Die zuvor beschriebene Stabilitätsuntersuchung wurde mit einer erfindungsgemäßen Lösung gemäß Beispiel 3 wiederholt. Die erhaltenen Ergebnisse sind in Tabelle 6 zusammengefaßt.

**Tabelle 6: Stabilitätsdaten einer erfindungsgemäßen hochkonzentrierten Ifosfamidlösung.**

| Zeit | Rel.Gehalt nach Lagerung 2-8°C | Rel.Gehalt nach Lagerung 15°C | Rel.Gehalt nach Lagerung 25°C | Rel.Gehalt nach Lagerung bei 40°C |
|---|---|---|---|---|
| 0 | 198,85 | - | 198,85 | 198,85 |
| | 100,0% | | 100,0% | 100,0% |
| | pH 7,6 | | pH 7,6 | pH 7,6 |
| 2 Monate | 192,30 | - | 187,12 | 66,39 |
| | 96,71% | | 94,10% | 33,39% |
| | pH 7,4 | | - | - |
| 4 Monate | 200,66 | - | 182,46 | 8,32 |
| | 100,91% | | 91,76% | 4,18% |
| | pH 7,4 | | pH 6,1 | pH 4,1 |
| 6 Monate | 195,51 | - | 170,67 | - |
| | 98,32% | | 85,83% | |
| | pH 7,4 | | pH 5,9 | |
| 9 Monate | 202,70 | - | 161,16 | |
| | 101,94% | | 81,05% | |
| | pH 7,3 | | pH 5,5 | |
| 12 Monate | 196,72 | - | - | - |
| | 98,92% | | | |
| | pH 7,3 | | | |
| 15 Monate | 192.27 | - | - | - |
| | 96.69% | | | |
| | pH 7,3 | | | |
| 18 Monate | 193,9 | - | - | - |
| | 97,51% | | | |
| | pH 7,3 | | | |

Es zeigt sich, daß selbst bei einer Lagerung über 18 Monate bei 2-8°C praktisch keine Zersetzung des Ifosfamids auftritt.

## Patentansprüche

1. Verwendung einer physiologisch verträglichen, in wässriger Lösung Chloridionen bildenden Verbindung zur Stabilisierung eines Oxazaphosphorins der allgemeinen Formel 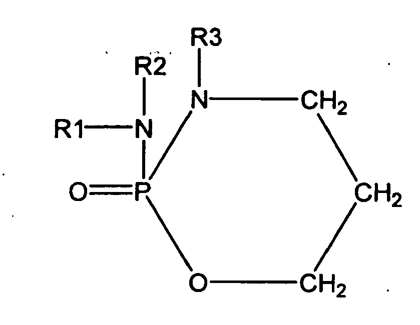 worin R1, R2 und R3 gleich oder verschieden sind und Wasserstoff, Methyl, Ethyl, 2-Chlorethyl oder 2-Methansulföriyloxyethyl bedeuten, mit der Maßgabe, dass R3 nicht Wasserstoff bedeutet, und dabei mindestens zwei dieser Reste 2-Chlorethyl und/oder 2-Methansulfonyloxyethyl sind, in wässrigen Arzneilösungen.

2. Verwendung nach Anspruch 1, wobei die Chloridionen bildende Verbindung Kochsalz ist.

3. Verwendung nach Anspruch 1 oder 2, wobei das Oxazaphosphorin Ifosfamid ist.

4. Verwendung nach einem der vorstehenden Ansprüche, wobei die Chloridionen bildende Verbindung in einer solchen Menge eingesetzt wird, dass pro Gramm Wirkstoff bis zu 1 g Chloridionen, bevorzugt 0,001 g bis 0,5 g Chloridionen, besonders bevorzugt 0,005 g bis 0,15 g Chloridionen in der Lösung enthaltend sind.

5. Arzneimittel in Form einer lagerstabilen, gebrauchsfertigen wässrigen, pyrogenfreien Lösung in einem abgedichteten Behälter, die nicht unmittelbar vor der Verwendung aus einer Trockensubstanz rekonstituiert wurde, umfassend mindestens einen Wirkstoff aus der Gruppe der Oxazaphosphorine der allgemeinen Formel 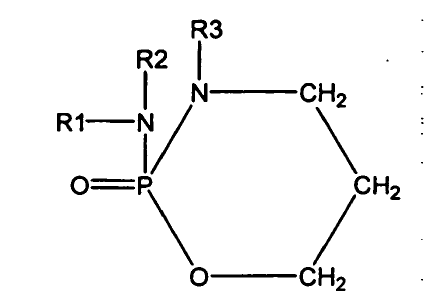 worin R1, R2 und R3 gleich oder verschieden sind und Wasserstoff, Methyl, Ethyl, 2-Chlorethyl oder 2-Methansulfonyloxyethyl bedeuten, mit der Maßgabe, dass R3 nicht Wasserstoff bedeutet, und dabei mindestens zwei dieser Reste 2-Chlorethyl und/oder 2-Methansuifonyloxyethyl sind, eine physiologisch verträgliche, in der wässrigen Lösung Chloridionen bildende Verbindung sowie gegebenenfalls mindestens einen lösungsvermittelnden Hilfsstoff und/oder mindestens eine Puffersubstanz.

6. Arzneimittel nach Anspruch 5, mit einem Wirkstoffgehalt von 0,1 g bis 50 g in 100 ml Lösung, vorzugsweise von 0,1 bis 30 g in 100 ml Lösung und insbesondere von 1 g bis 20 g in 100 ml Lösung.

7. Arzneimittel nach Anspruch 5 oder 6, wobei der Wirkstoff Ifosfamid ist.

8. Arzneimittel nach einem der Ansprüche 5 bis 7, wobei die Chloridionen bildende Verbindung Kochsalz ist und in einer Menge von bis zu 1 g/g Wirkstoff, bevorzugt von 0,001 g/g Wirkstoff bis 0,25 g/g Wirkstoff, besonders bevorzugt von 0,01 g/g Wirkstoff bis 0,15 g/g Wirkstoff enthalten ist.

9. Arzneimittel nach einem der Ansprüche 5 bis 8, wobei der pH-Wert der Lösung gegebenenfalls durch Zusatz von Puffersubstanzen und/oder von Ansäuerungs- oder Alkalisierungsmittel auf 4,5 bis 10,0 eingestellt worden ist.

10. Arzneimittel nach einem der Ansprüche 5 bis 9, wobei der pH-Wert auf 6,5 bis 9,5, vorzugsweise auf 7,5 bis 9,5 und besonders bevorzugt auf 7,3 bis 8,5 eingestellt ist.

11. Arzneimittel nach einem der Ansprüche 5 bis 10, die als lösungsvermittelnden Hilfsstoff Harnstoff in einer Konzentration von 0, 1 g bis 50 g in 100 ml Lösung enthält.

12. Arzneimittel nach Anspruch 11, die Harnstoff in einer Konzentration von 0,5 g bis 30 g in 100 ml Lösung und insbesondere von 1,0 g bis 20 g in 100 ml Lösung enthält.

13. Arzneimittel nach einem der Ansprüche 5-12, umfassend
40 mg/ml Ifosfamid
30 mg/ml Harnstoff
6,25 mg/ml di-Natriumhydrogenphosphat-Dihydrat
0,8 mg/ml Natriumchlorid
sowie Salzsäure zur Einstellung des pH-Werts und Wasser.

14. Arzneimittel nach einem der Ansprüche 5 bis 12, umfassend
200 mg/ml Ifosfamid
150 mg/ml Harnstoff
15 mg/ml di-Natriumhydrogenphosphat-Dihydrat
4,0 mg/ml Natriumchlorid
sowie Salzsäure zur Einstellung des pH-Werts und Wasser.

15. Arzneimittel nach einem der Ansprüche 5 bis 12, die zusätzlich Ethanol in einer Menge von bis zu 79 Vol.-%, vorzugsweise von bis zu 49 Vol.-% und besonders bevorzugt von 5 bis 30 Vol.-% enthält.

16. Arzneimittel nach einem der Ansprüche 5 bis 15, die zusätzlich ein Uroprotectivum enthält.

17. Verfahren zur Herstellung einer Arzneilösung nach einem der Ansprüche 5 bis 16, bei dem die Komponenten in beliebiger Reihenfolge in Wasser gelöst werden.

## Claims

1. Use of a physiologically acceptable compound that forms chloride ions in aqueous solution for stabilising an oxazaphosphorine of the general formula 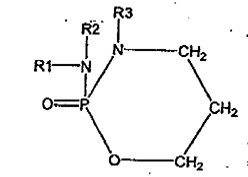 wherein R1, R2 and R3 are the same or different and represent hydrogen, methyl, ethyl, 2-chloroethyl or 2-methanesulfonyloxyethyl, with the proviso that R3 does not represent hydrogen and at least two of those radicals are 2-chloroethyl and/or 2-methanesulfonyloxyethyl, in aqueous medicament solutions.

2. Use according to claim 1, wherein the compound that forms chloride ions is common salt.

3. Use according to claim 1 or 2, wherein the oxazaphosphorine is ifosfamide.

4. Use according to any one of the preceding claims, wherein the compound that forms chloride ions is used in an amount such that up to 1 g of chloride ions, preferably from 0.001 g to 0.5 g of chloride ions, particularly preferably from 0.005 g to 0.15 g of chloride ions, is present in the solution per gram of active ingredient.

5. Medicament in the form of a storage-stable, ready-for-use, aqueous, pyrogen-free solution in a sealed container, which solution has been reconstituted from a dry substance not immediately before use, comprising at least one active ingredient from the group of the oxazaphosphorines of the general formula 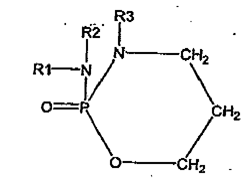 wherein R1, R2 and R3 are the same or different and represent hydrogen, methyl, ethyl, 2-chloroethyl or 2-methanesulfonyloxyethyl, with the proviso that R3 does not represent hydrogen and at least two of those radicals are 2-chloroethyl and/or 2-methanesulfonyloxyethyl, a physiologically acceptable compound that forms chloride ions in the aqueous solution, as well as, optionally, at least one solubilising auxiliary substance and/or at least one buffer substance.

6. Medicament according to claim 5 having an active ingredient content of from 0.1 g to 50 g in 100 ml of solution, preferably from 0.1 to 30 g in 100 ml of solution and in particular from 1 g to 20 g in 100 ml of solution.

7. Medicament according to claim 5 or 6, wherein the active ingredient is ifosfamide.

8. Medicament according to any one of claims 5 to 7, wherein the compound that forms chloride ions is common salt and is present in an amount of up to 1 g/g of active ingredient, preferably from 0.001 g/g of active ingredient to 0.25 g/g of active ingredient, particularly preferably from 0.01 g/g of active ingredient to 0.15 g/g of active ingredient.

9. Medicament according to any one of claims 5 to 8, wherein the pH value of the solution has optionally been adjusted to from 4.5 to 10.0 by addition of buffer substances and/or acidifying or alkalising agents.

10. Medicament according to any one of claims 5 to 9, wherein the pH value has been adjusted to from 6.5 to 9.5, preferably from 7.5 to 9.5 and particularly preferably from 7.3 to 8.5.

11. Medicament according to any one of claims 5 to 10, comprising as the solubilising auxiliary substance urea in a concentration of from 0.1 g to 50 g in 100 ml of solution.

12. Medicament according to claim 11, comprising urea in a concentration of from 0.5 g to 30 g in 100 ml of solution and in particular from 1.0 g of 20 g in 100 ml of solution.

13. Medicament according to any one of claims 5 to 12, comprising
40 mg/ml ifosfamide
30 mg/ml urea
6.25 mg/ml disodium hydrogen phosphate dihydrate
0.8 mg/ml sodium chloride
as well as hydrochloric acid for adjusting the pH value, and water.

14. Medicament according to any one of claims 5 to 12, comprising
200 mg/ml ifosfamide
150 mg/ml urea
15 mg/ml disodium hydrogen phosphate dihydrate
4.0 mg/ml sodium chloride
as well as hydrochloric acid for adjusting the pH value, and water.

15. Medicament according to any one of claims 5 to 12, additionally comprising ethanol in an amount of up to 79 vol.%, preferably up to 49 vol.% and particularly preferably from 5 to 30 vol.%.

16. Medicament according to any one of claims 5 to 15, additionally comprising a uroprotective agent.

17. Process for the preparation of a medicament solution according to any one of claims 5 to 16, in which the components are dissolved in any desired sequence in water.

## Revendications

1. Utilisation d'un composé physiologiquement acceptable, qui forme des ions chlorure en solution aqueuse, pour stabiliser une oxazaphosphorine de formule générale : 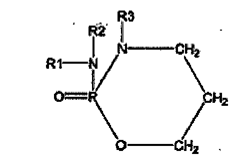 dans laquelle R1, R2 et R3 peuvent être identiques ou différents et représentent un hydrogène, un méthyle, un éthyle, un 2-chloroéthyle ou un 2-méthanesulfonyloxyéthyle, avec la condition que R3 ne représente pas un hydrogène, au moins deux de ces groupes représentant un 2-chloroéthyle et/ou un 2-méthanesulfonyloxyéthyle, dans des solutions médicamenteuses aqueuses.

2. Utilisation selon la revendication 1, dans laquelle le composé qui forme des ions chlorure est le sel de cuisine.

3. Utilisation selon la revendication 1 ou 2, dans laquelle l'oxazaphosphorine est l'ifosfamide.

4. Utilisation selon l'une des revendications précédentes, dans laquelle le composé qui forme des ions chlorure est utilisé en une quantité telle que, par gramme de substance active, la solution contient jusque 1 g d'ions chlorure, de préférence 0,001 g à 0,5 g d'ions chlorure et plus préférablement 0,005 g à 0,15 g d'ions chlorure.

5. Médicament sous la forme d'une solution aqueuse exempte de pyrogène, prête à l'emploi et stable au stockage, dans un récipient étanche, qui n'a pas été reconstituée à partir d'une substance sèche immédiatement avant son utilisation et qui comprend au moins une substance active sélectionnée dans le groupe des oxazaphosphorines de formule générale : 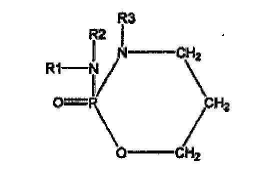 dans laquelle R1, R2 et R3 peuvent être identiques ou différents et représentent un hydrogène, un méthyle, un éthyle, un 2-chloroéthyle ou un 2-méthanesulfonyloxyéthyle, avec la condition que R3 ne représente pas un hydrogène, au moins deux de ces groupes représentant un 2-chloroéthyle et/ou un 2-méthanesulfonyloxyéthyle, un composé physiologiquement acceptable, qui forme des ions chlorure en solution aqueuse, ainsi qu'éventuellement au moins une substance auxiliaire de solubilisation et/ou au moins une substance tampon.

6. Médicament selon la revendication 5, dont la teneur en substance active est de 0,1 g à 50 g dans 100 ml de solution, de préférence de 0,1 à 30 g dans 100 ml de solution et en particulier de 1 g à 20 g dans 100 ml de solution.

7. Médicament selon la revendication 5 ou 6, dans lequel la substance active est l'ifosfamide.

8. Médicament selon l'une des revendications 5 à 7, dans lequel le composé qui forme des ions chlorure est le sel de cuisine et est contenu en une quantité allant jusque 1 g/g de substance active, de préférence de 0,001 g/g de substance active à 0,25 g/g de substance active et plus préférablement de 0,01 g/g de substance active à 0,15 g/g de substance active.

9. Médicament selon l'une des revendications 5 à 8, dans lequel la valeur du pH de la solution a éventuellement été ajustée entre 4,5 et 10,0, par addition de substances tampons et/ou d'agents d'acidification ou d'alcalinisation.

10. Médicament selon l'une des revendications 5 à 9, dans lequel dans lequel la valeur du pH est ajustée entre 6,5 et 9,5, de préférence entre 7,5 et 9,5 et de plus préférablement entre 7,3 et 8,5.

11. Médicament selon l'une des revendications 5 à 10, qui contient comme substance auxiliaire de solubilisation de l'urée à une concentration allant de 0,1 g à 50 g dans 100 ml de solution.

12. Médicament selon la revendication 11, qui contient de l'urée à une concentration de 0,5 g à 30 g dans 100 ml de solution et en particulier de 1,0 g à 20 g dans 100 ml de solution.

13. Médicament selon l'une des revendications 5 à 12, qui comprend :
40 mg/ml d'ifosfamide,
30 mg/ml d'urée,
6,25 mg/ml de dihydrogénophosphate de sodium dihydraté,
0,8 mg/ml de chlorure de sodium
ainsi que de l'acide chlorhydrique pour l'ajustement de la valeur du pH, et de l'eau.

14. Médicament selon l'une des revendications 5 à 12, qui comprend:
200 mg/ml d'ifosfamide,
150 mg/ml d'urée,
15 mg/ml de dihydrogénophosphate de sodium dihydraté,
4,0 mg/ml de chlorure de sodium
ainsi que de l'acide chlorhydrique pour l'ajustement de la valeur du pH, et de l'eau.

15. Médicament selon l'une des revendications 5 à 12, qui comprend en outre de l'éthanol en quantité allant jusque 79 % en volume, de préférence jusque 49 % en volume et plus préférablement de 5 à 30 % en volume.

16. Médicament selon l'une des revendications 5 à 15, qui comprend en outre un "uroprotectivum".

17. Procédé de préparation d'une solution médicamenteuse selon l'une des revendications 5 à 16, dans lequel les composants sont mélangés à de l'eau dans n'importe quel ordre.
